# EUROPEAN PATENT SPECIFICATION

(11) **EP 2 719 418 B1**
(45) Date of publication and mention of the grant of the patent: **23.10.2024**
(21) Application number: 12796386.6
(22) Date of filing: 07.06.2012
(51) Int. Cl.: A61M 25/10, A61M 25/00

(54) **BALLOON CATHETER AND METHOD FOR PRODUCING SAME**
BALLONKATHETER UND VERFAHREN ZU SEINER HERSTELLUNG
CATHÉTER À BALLONNET ET PROCÉDÉ DE PRODUCTION DE CELUI-CI

(30) Priority: 08.06.2011 JP 2011128665
(43) Date of publication of application: 16.04.2014
(73) Proprietor: Kaneka Corporation, Osaka-shi, Osaka 530-8288 (JP)
(72) Inventor: MIZOKAMI Yasuhiro, Settsu-shi Osaka 566-0072 (JP)
(74) Representative: TBK
(86) International application number: PCT/JP2012/064701
(87) International publication number: WO 2012/169593

(56) References cited:
- WO-A1-2010/067875
- WO-A1-2010/067875
- WO-A1-2012/116337
- WO-A2-97/44082
- JP-A- 2001 299 921
- JP-A- 2002 143 314
- JP-A- 2007 082 802
- JP-A- 2008 173 137
- US-A1- 2006 030 835
- US-A1- 2010 057 018
- US-B1- 6 960 186
- US-B1- 8 034 045

## Description

### Technical Field

The present invention generally relates to a balloon catheter used in percutaneous transluminal coronary angioplasty (PTCA). More specifically, the present invention relates to a perfusion balloon catheter that makes it possible to inflate a balloon inside a blood vessel for a long period of time.

### Background Art

Conventionally, percutaneous angioplasty has been widely utilized in dilation treatment for a narrow or occluded part of a vascular lumen for the purpose of recovering or improving blood flow in a coronary artery or a peripheral blood vessel. An example of a balloon catheter used in percutaneous angioplasty will be given below. In a common structure of the balloon catheter, a balloon that can be freely inflated and deflated by adjusting the internal pressure is joined to the end of a shaft, and a lumen into which a guide wire is inserted (guide wire lumen) and a lumen through which pressure fluid for adjusting the internal pressure of the balloon is supplied (inflation lumen) are provided inside the shaft along the longitudinal direction of the shaft. Further, a balloon catheter includes an over-the-wire balloon catheter in which the guide wire lumen extends throughout the entire length thereof, and a rapid exchange balloon catheter in which the guide wire lumen extends only in the distal part of the catheter in order to allow easy exchange of the balloon catheter.

A common surgical case of PTCA using such a balloon catheter is as follows. First, a guide catheter is inserted from a puncture part in a femoral artery, a brachial artery, a radial artery or the like so as to pass through the main artery, and the distal end of the guide catheter is positioned on an entrance of a coronary artery. Then, a guide wire which has been inserted into the guide wire lumen is advanced beyond a narrow part of the coronary artery, and a balloon catheter is inserted along the guide wire so that the position of a balloon is matched with the position of the narrow part. Then, pressure fluid is supplied to the balloon through the inflation lumen using a device such as an indeflator to inflate the balloon, thereby performing dilation treatment on the narrow part. After the dilation treatment is performed on the narrow part, the balloon is deflated by reducing the pressure inside thereof and removed from the body, thereby finishing the PTCA.

In balloon catheters used in PTCA, there is a classification called a perfusion balloon catheter in addition to a general balloon catheter. In a perfusion balloon catheter, blood can be perfused from the proximal side of a balloon to the distal side thereof with the balloon inflated inside a blood vessel in a narrow part or the like. A perfusion balloon catheter generally has a proximal side perfusion port on a shaft at the proximal side of the proximal end of a balloon and a distal side perfusion port at the distal side of the distal end of the balloon, and can perfuse blood through the perfusion ports and a lumen which perfuses blood inside thereof (also referred to as a perfusion lumen). The perfusion balloon catheter is disclosed in Patent Documents 1 and 2. Since an ischemic state of a blood vessel at a more distal position than the balloon can be prevented or minimized by performing blood perfusion even when the balloon is in an inflated state, the balloon inflation can be maintained for a long period of time.

As disclosed in Patent Documents 1 and 2, the perfusion lumen is generally formed to pass through an inside tube which passes through the inside of the balloon. In order to perfuse a larger amount of blood, a perfusion lumen having a larger cross sectional area is advantageous. Therefore, a perfusion balloon catheter has a larger inside tube than a general balloon catheter. On the other hand, since pressure is applied to the inside tube which passes through the inside of the balloon from the outside thereof when inflating the balloon, the larger the inside tube is, the larger stress applied to the outer wall of the inside tube becomes. As a result, the inside tube tends to be easily crushed or kinked. Therefore, an inside tube of a perfusion balloon catheter is made more rigid than that of a general balloon catheter or reinforced with metal.

Recently, a polyamide or polyurethane thermoplastic polymer has often been used as the material for a balloon of a balloon catheter used in PTCAin view of flexibility, strength, formability, processability and the like. Further, when the inside tube of the balloon catheter is formed of a material that is different from the material of the balloon, it is necessary to bond the inside tube and the balloon to each other with adhesive, and the bonded part thereby disadvantageously becomes rigid. Therefore, it has become common that at least an outer layer of the inside tube is formed of the same kind of material as that of the balloon, and the outer layer is heat-welded to the balloon. Thus, it is necessary that the inside tube of the perfusion balloon catheter be reinforced with metal or the like, and at least the outer layer of the inside tube be formed of a thermoplastic polymer such as a polyamide material.

Patent Documents 3 and 4 disclose a method for reinforcing a catheter shaft with metal using a coil structure or a braid structure. Both of Patent Documents 3 and 4 disclose a method in which a reinforcing metal (a reinforcing coil in Patent Document 3 and a metal braid in Patent Document 4) is applied around an inner layer tube which is formed of a fluorine-containing ethylene polymer having excellent wettability such as polytetrafluoroethylene, and an outer layer tube which is formed of a polyamide material or the like is coated thereon. Recently, a thermoplastic polymer such as a polyamide material has often been used as the material of a catheter shaft in view of flexibility, strength, and processability. However, since it is generally necessary, when forming a composite tube, to previously insert a lumen forming core member into an inner layer tube and remove the core member therefrom after forming the tube, it has been necessary to use a material having excellent wettability in the inner layer tube. For example, when a thermoplastic polymer is used in the inner layer, there is caused a problem such that the inner layer is stuck to the surface of the lumen forming core member, and the lumen forming core member cannot therefore be pulled out. On the other hand, a material having excellent wettability such as a fluorine-containing ethylene polymer has a low affinity for a polyamide material. Therefore, the inner layer and the outer layer are delaminated from each other in such a configuration. If the inner layer is delaminated from the outer layer inside the body, the inner layer may remain therein. In some cases, the remaining inner layer may threaten patient's life. In Patent Documents 3 and 4, chemical etching is performed on or a reactive functional group is introduced into the outer surface of the inner layer tube formed of a fluorine-containing ethylene polymer or the like to thereby improve the adhesiveness between the inner layer tube and the outer layer tube. However, it is necessary to introduce a large-scale apparatus to perform the surface processing, and there is a limit to improving the adhesiveness by the surface processing. Therefore, the possibility of delamination of the inner layer from the outer layer could not be completely eliminated. Especially in a balloon catheter, a balloon is inflated by pressure so as to be stretched in the radial direction and the axial direction. Since an inside tube is also stretched in the axial direction along with the extension of the balloon, delamination of an inner layer disadvantageously occurs more easily.
WO 97 / 44 082 A2 discloses a catheter for use in an emboli containment system. The catheter has a tubular body with a metallic braid construction. Two lumen extend through the tubular body, the lumen being in a side-by-side configuration. One of the lumen functions as an inflation lumen, and is in fluid communication with an inflatable balloon mounted on the distal end of the catheter. The second lumen is adapted to receive other therapeutic catheters which comprise the emboli containment system.
WO 2010 / 067 875 A1 discloses a medical tube which is flexible and achieves good kink resistant properties and high tensile strength at the same time. Even when connected to another tube, the medical tube maintains the above-mentioned kink resistant properties and tensile strength. A medical tube is provided with at least one coil layer, a first outer layer provided outside the coil layer and a second outer layer provided outside the first outer layer. The melting point of the material which forms the second outer layer is lower than the melting point of the material forming the first outer layer. The outer surface of the coil layer and the inner surface of the first outer layer are in contact with and affixed to each other in a slidable state.
Patent Documents 5 to 8 disclose further prior art.

### Citation List

### Patent Literatures

Patent Document 1: Japanese Patent JP 3 382 966 B2
Patent Document 2: JP H09 - 507 391 A
Patent Document 3: JP H05 - 192 411 A
Patent Document 4: JP 2007 - 000 392 A
Patent Document 5: US 2006 / 030 835 A1
Patent Document 6: US 2010 / 057 018 A1
Patent Document 7: WO 2012 / 116 337 A1 (post-published)
Patent Document 8: US 8 034 045 B1

### Summary of Invention

### Technical Problem

In view of the above problems, an object of the present invention is to provide a balloon catheter having excellent productivity, the balloon catheter being capable of, in a balloon inside tube of the balloon catheter having an inner layer, an outer layer, and a support body, preventing delamination of the inner layer when a guide wire is inserted into the balloon inside tube and also easily removing a lumen forming core member after forming the balloon inside tube during a production process when a balloon is heat-welded to form an end part of the balloon.

Further, another object of the present invention is to provide a method for efficiently producing the balloon catheter provided with the balloon inside tube.

### Solution to Problem

As a result of intensive studies to solve the above problems, the present inventor has focused on the melting points of the inner and the outer layers forming the balloon inside tube

The above-mentioned problems are solved by a balloon catheter according to claim 1.

The above-mentioned problems are further solved by a method for producing a balloon catheter according to claim 15.
Further advantageous embodiments are disclosed in the dependent claims.

### Advantageous Effects of Invention

According to the balloon catheter of the present invention, the balloon, and the inner and outer layers forming the balloon inside tube are heat-welded to each other. Therefore, delamination of the inner layer of the balloon inside tube can be prevented.

In an advantageous embodiment of the present invention, since the heat-welding is performed at a temperature that is equal to or higher than the melting point of the thermoplastic polymer forming the outer layer of the balloon inside tube as well as lower than the melting point of the thermoplastic polymer forming the inner layer, the outer layer is melted without melting the inner layer.

Therefore, the lumen forming core member can be easily removed after forming the balloon inside tube which is formed of thermoplastic polymers, and delamination of the inner layer does not easily occur even when a guide wire makes contact with the inner layer such as a case where the guide wire is inserted into and removed from the inner lumen of the balloon inside tube.

In the present invention, the balloon has a distal side sleeve at the distal side thereof, and the distal side sleeve is heat-welded to a distal part of the balloon inside tube at a temperature equal to or higher than the melting point of the thermoplastic polymer forming the inner layer of the balloon inside tube. Therefore, the distal side sleeve and the outer and inner layers of the balloon inside tube are integrated with each other, thereby making it possible to further suppress the delamination of the inner layer of the balloon inside tube.

In an advantageous embodiment of the present invention, the balloon has a proximal side sleeve at the proximal side thereof, and the proximal side sleeve is heat-welded to a proximal part of the balloon inside tube at a temperature equal to or higher than the melting point of the thermoplastic polymer forming the inner layer of the balloon inside tube. Therefore, the sleeves of the balloon can be firmly welded to the balloon inside tube.

In an advantageous embodiment of the present invention, each of the thermoplastic polymers is polyamide or a polyamide elastomer. Therefore, the balloon catheter has adequate flexibility, strength, and processability.

Further, in an advantageous embodiment of the present invention, the Shore D hardness of the inner layer is in the range of 63D to 74D. Therefore, when the balloon and the balloon inside tube are heat-welded to each other, more firm welding at the interfaces between the inner layer, the outer layer, and the balloon tends to be achieved.

In an advantageous embodiment of the present invention, the shore D hardness of the outer layer is in the range of 40D to 55D. Therefore, the balloon catheter has more adequate flexibility.

In an advantageous embodiment of the present invention, the support body is embedded in the outer layer, extends at least over a part of the balloon inside tube, the part being positioned within the inner lumen of the balloon, and extends throughout the entire circumferential direction of the balloon inside tube. Therefore, it is possible to improve the pressing performance and the buckling strength in the radial direction of the balloon catheter.

In an advantageous embodiment of the present invention, the support body is a coil-shaped support body. Therefore, it is possible to improve the buckling strength in the radial direction of the balloon portion and impart pliability to the balloon inside tube.

In an advantageous embodiment of the present invention, the coil-shaped body is formed of a rectangular wire. Therefore, improvement of the pressing performance in the axial direction of the balloon catheter can be expected.

In an advantageous embodiment of the present invention, the coil-shaped support body is formed of a round wire. Therefore, improvement of the pliability of the balloon catheter can be expected.

In an advantageous embodiment of the present invention, the balloon catheter includes a radiopaque marker located within the balloon inside tube. Therefore, it is possible to confirm the position of the balloon inserted into the body.

In an advantageous embodiment of the present invention, the radiopaque marker is located at more inner position in the radial direction than the support body. Therefore, it is possible to reduce deviation of the support body in the axial direction when forming the balloon inside tube.

Further, it is preferred that the balloon inside tube be connected to a distal end of a distal side shaft, a proximal side shaft be connected to a proximal end of the distal side shaft, and a hub be attached to a proximal part of the proximal side shaft.

In advantageous embodiment of the present invention, the balloon catheter is a perfusion balloon catheter, and an inner lumen of the balloon inside tube forms a perfusion lumen. Therefore, the perfusion lumen and the guide wire lumen can be formed as a single lumen.

### Brief Description of the Drawings

Fig. 1 is an outline drawing of a rapid exchange balloon catheter A as an embodiment of a balloon catheter according to the present invention.
Fig. 2 is a cross-sectional view taken along line X-X' of Fig. 1 in an embodiment having a coaxial distal side shaft 4.
Fig. 3 is a cross-sectional view taken along line X-X' of Fig. 1 in an embodiment having a biaxial distal side shaft 4.
Fig. 4 is a cross-sectional view taken along line X-X' of Fig. 1 in another embodiment having a biaxial distal side shaft 4.
Fig. 5 is a schematic view illustrating a state where a common perfusion balloon catheter A is inserted into a blood vessel 15.
Fig. 6 is a schematic view illustrating a state where a balloon 5 of the common perfusion balloon catheter A is inflated inside the blood vessel 15.
Fig. 7 is an axial direction cross-sectional view of the perfusion balloon catheter A according to the present invention from the distal side shaft 4 through the balloon 5.
Fig. 8 is an axial direction cross-sectional view of a perfusion balloon catheter A' of another embodiment according to the present invention from a distal side shaft 4 through a balloon 5.
Fig. 9 is an outline drawing of the perfusion balloon catheter A according to the present invention near distal side perfusion ports 13.
Fig. 10 is an axial direction cross-sectional view in an embodiment of a balloon inside tube 20 according to the present invention.
Fig. 11 is a front view in an embodiment of a support body 23 of the balloon inside tube 20 according to the present invention.
Fig. 12 is a side view in an embodiment of the support body 23 of the balloon inside tube 20 according to the present invention
Fig. 13 is an axial direction cross-sectional view of the balloon catheter A according to the present invention in which the balloon 5 is disposed on the surface of the balloon inside tube 20.
Fig. 14 is a front view of a lumen forming core member 27 inserted into an inner tube 26 in a production process of an embodiment of the balloon inside tube 20 according to the present invention.
Fig. 15 is an axial direction cross-sectional view of the lumen forming core member 27 inserted into the inner tube 26 in the production process of the embodiment of the balloon inside tube 20 according to the present invention.
Fig. 16 is a radial direction cross-sectional view of the lumen forming core member 27 inserted into the inner tube 26 in the production process of the embodiment of the balloon inside tube 20 according to the present invention.
Fig. 17 is a front view of radiopaque markers 24 attached to the outside of the inner tube 26 in the production process of the embodiment of the balloon inside tube 20 according to the present invention.
Fig. 18 is an axial direction cross-sectional view of the radiopaque markers 24 attached to the outside of the inner tube 26 in the production process of the embodiment of the balloon inside tube 20 according to the present invention.
Fig. 19 is a radial direction cross-sectional view of a portion in which a radiopaque marker 24 is attached to the outside of the inner tube 26 in the production process of the embodiment of the balloon inside tube 20 according to the present invention.
Fig. 20 is a front view of the support body 23 disposed on the outside of the inner tube 26 in the production process of the embodiment of the balloon inside tube 20 according to the present invention.
Fig. 21 is an axial direction cross-sectional view of the support body 23 disposed on the outside of the inner tube 26 in the production process of the embodiment of the balloon inside tube 20 according to the present invention.
Fig. 22 is a radial direction cross-sectional view of a portion that includes the support body 23 disposed on the outside of the inner tube 26 and the radiopaque marker 24 in the production process of the embodiment of the balloon inside tube 20 according to the present invention.
Fig. 23 is a front view of an outer tube 28 disposed so as to cover the support body 23 in the production process of the embodiment of the balloon inside tube 20 according to the present invention.
Fig. 24 is an axial direction cross-sectional view of the outer tube 28 disposed so as to cover the support body 23 in the production process of the embodiment of the balloon inside tube 20 according to the present invention.
Fig. 25 is a radial direction cross-sectional view of a portion that includes the outer tube 28 disposed so as to cover the support body 23, the support body 23, and the radiopaque marker 24 in the production process of the embodiment of the balloon inside tube 20 according to the present invention.
Fig. 26 is a front view of a heat-shrinkable tube 29 disposed so as to cover the outer tube 28 in the production process of the embodiment of the balloon inside tube 20 according to the present invention.
Fig. 27 is an axial direction cross-sectional view of the heat-shrinkable tube 29 disposed so as to cover the outer tube 28 in the production process of the embodiment of the balloon inside tube 20 according to the present invention.
Fig. 28 is a radial direction cross-sectional view of a portion that includes the heat-shrinkable tube 29 disposed so as to cover the outer tube 28, the support body 23, and the radiopaque marker 24 in the production process of the embodiment of the balloon inside tube 20 according to the present invention.

### Description of Embodiments

Hereinbelow, various embodiments of a balloon catheter according to the present invention will be described in detail with reference to the drawings. The balloon catheter according to the present invention can be applied, without limitation, to a general balloon catheter, a perfusion balloon catheter, a drug-releasing balloon catheter and the like. However, since application to a perfusion balloon catheter is particularly useful, a perfusion balloon catheter will be described below as a main embodiment.

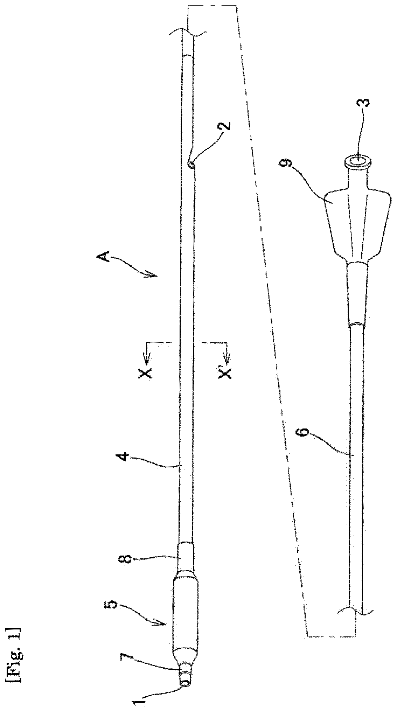

As shown in Fig. 1, a balloon catheter A of the present invention includes a distal side shaft 4, a balloon 5 which is attached to a distal part of the distal side shaft 4, a proximal side shaft 6, and a hub 9 which is attached to a proximal part of the proximal side shaft 6. Although the embodiments of the present invention will be described on the basis of the rapid exchange balloon catheter as shown in Fig. 1, the preset invention can also be applied to an over-the-wire balloon catheter, an on-the-wire balloon catheter provided with a guide wire, and the like.

In the case of the rapid exchange balloon catheter, the proximal side shaft 6 is required to be a rigid shaft. Therefore, the proximal side shaft 6 can be a metal tube, or a core wire can be provided inside the shaft. As shown in Fig. 1, a proximal side guide wire port 2 can be provided near the junction between the proximal side shaft 6 and the distal side shaft 4, and a distal side guide wire port 1 can be provided at the tip of the catheter. A guide wire lumen through which a guide wire passes can be provided from the proximal side guide wire port 2 up to the distal side guide wire port 1.

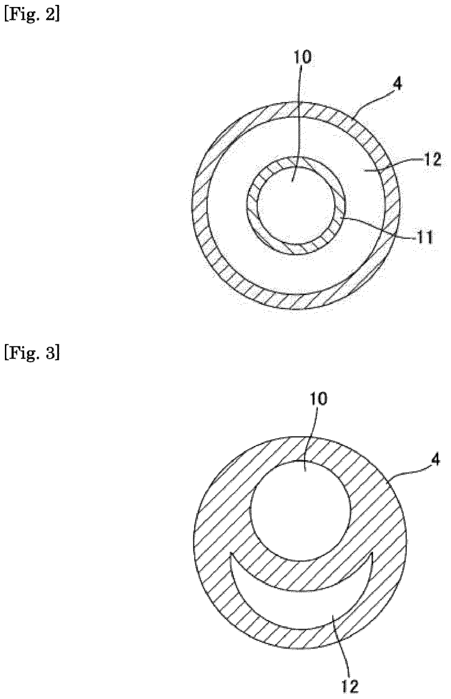

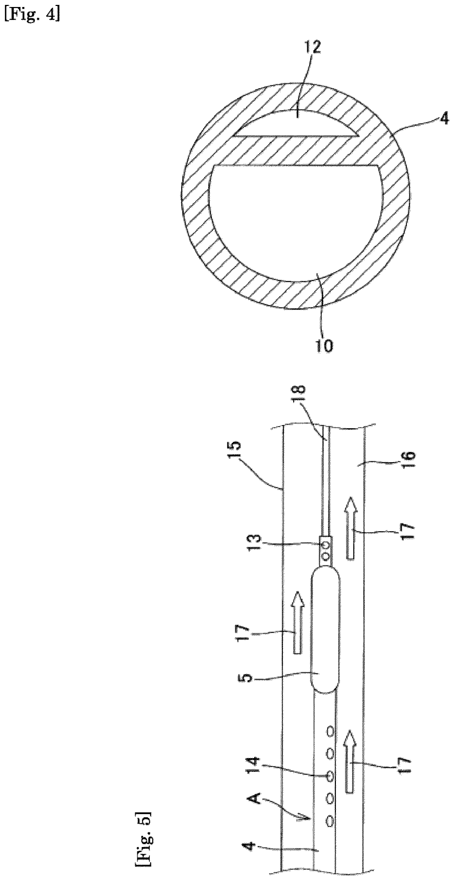

Further, in order to inflate the balloon 5, an inflation lumen 12 which communicates with the inside of the balloon 5 can be provided in parallel to the guide wire lumen 10 from an inflation port 3 at the proximal end of the hub 9 through the inside of the proximal side shaft 6 and the distal side shaft 4. The guide wire lumen 10 and the inflation lumen 12 may form a coaxial configuration (double-tube structure) as shown in Fig. 2, or may also form a biaxial configuration (parallel structure) as shown in Figs. 3 and 4. For example, in the rapid exchange balloon catheter A as shown in Fig. 1, since a guide wire is inserted from the proximal side guide wire port 2, the guide wire lumen 10 extends only within the distal side shaft 4. Therefore, only the distal side shaft 4 can be formed into a coaxial or biaxial shaft. As shown in Fig. 2, the guide wire lumen 10 of the coaxial distal side shaft 4 is formed by an inside tube 11.

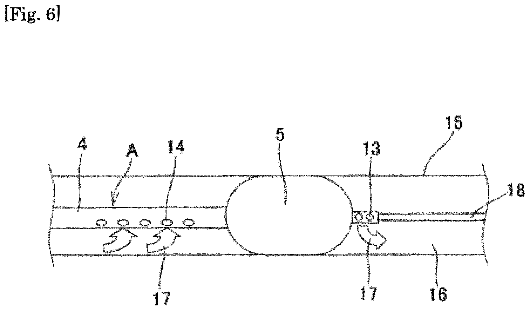

Generally, the perfusion balloon catheter A is advanced into the blood vessel 15 along a guide wire 18 as shown in Fig. 5. When the blood vessel 15 is a coronary artery, the perfusion balloon catheter A is inserted into the blood vessel 15 from the proximal side of the perfusion balloon catheter A toward the distal side thereof along a flowing direction 17 of blood 16. Then, as shown in Fig. 6, in order for the perfusion balloon catheter A to have a function to perfuse the blood 16 from the proximal side of the balloon 5 toward the distal side thereof when the balloon 5 is inflated inside the blood vessel 15, proximal side perfusion ports 14 can be provided on the distal side shaft 4 at more proximal positions than the balloon 5, and distal side perfusion ports 13 can be provided on the distal side shaft 4 at more distal positions than the balloon 5 so that the blood 16 flows inside the blood vessel 15 regardless of the existence of the balloon 5.

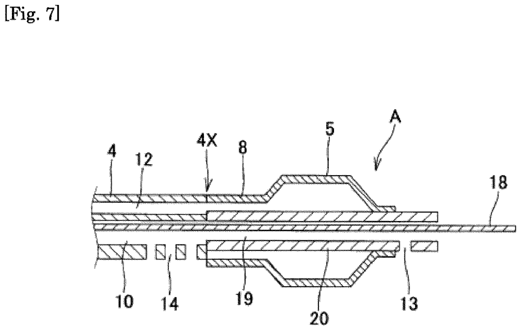

The balloon catheter A of the present invention has the same configuration as a perfusion balloon catheter as described above. In particular, a perfusion lumen 19 for passing blood therethrough is formed by the balloon inside tube 20 which passes through the inner lumen of the balloon 5. For example, as shown in Fig. 7, the proximal end of the balloon inside tube 20 is brought to butt against and bonded to a distal end 4X of the distal side shaft 4, and the perfusion lumen 19 is provided so that blood passes from the proximal side perfusion ports 14 formed on the distal side shaft 4 toward the distal side perfusion ports 13 formed on the balloon inside tube 20. The perfusion lumen 19 and the guide wire lumen 10 can be formed as a single lumen. When the perfusion lumen 19 and the guide wire lumen 10 are formed as a single lumen in this manner, the proximal side perfusion ports 14 are only required to be formed on the wall of the distal side shaft 4 so as to communicate with the guide wire lumen 10 of the distal side shaft 4.

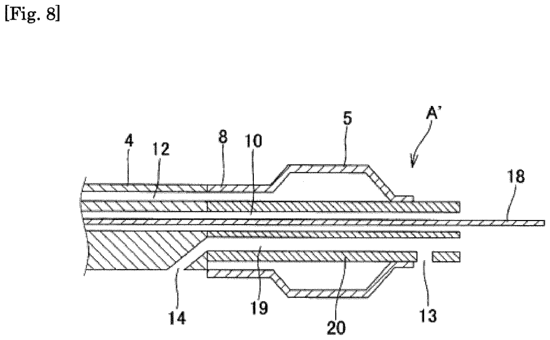

Further, in order to increase the perfusion amount of the blood 16 even when the balloon 5 is in an inflated state as shown in Fig. 6, an additional lumen other than the guide wire lumen 10 can also be provided in the balloon inside tube 20 as in a balloon catheter A' shown in Fig. 8. The number of lumens provided in the balloon inside tube 20 may, for example, be two as in the balloon catheter A' shown in Fig. 8, or three or more as necessary. When multiple lumens are provided in this manner, the cross-sectional shape of the balloon inside tube 20 in the radial direction may, for example, have a biaxial structure in which the guide wire lumen and the perfusion lumen are arranged in parallel to each other. However, a coaxial structure and other structures are also possible.

In the present invention, when forming the proximal side perfusion ports 14, a part of the distal side shaft 4, the part being positioned immediately near the balloon 5 at the proximal side thereof, preferably has a biaxial structure that has at least the inflation lumen 12 and the guide wire lumen 10 in view of easy processing. The inflation lumen 12 of the biaxial distal side shaft 4 communicates with the inner lumen of the balloon 5 so that the balloon 5 can be inflated and deflated in accordance with a conventional method. On the other hand, the guide wire lumen 10 communicates with the inner lumen of the balloon inside tube 20.

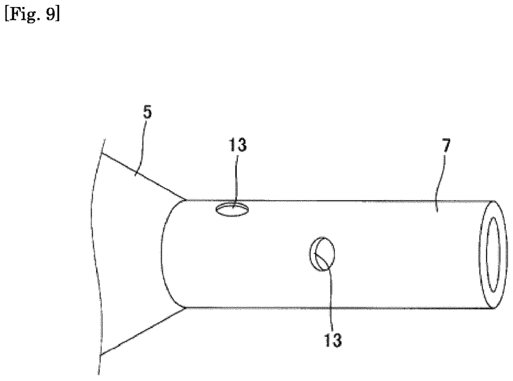

Each of the perfusion ports 13 and 14 may have any size as long as it is sufficient for the perfusion of the blood 16. Further, each of the number of the perfusion ports 13 and the number of the perfusion ports 14 may be one, or may also be two or more. However, given the possibility of blockage of the perfusion ports 13 and 14 due to a thrombus or the like, each of the number of the perfusion ports 13 and the number of the perfusion ports 14 is preferably two or more in view of safety. The distal side perfusion ports 13 may be disposed on a straight line, or may also be disposed by shifting one another in the circumferential direction, and the proximal side perfusion ports 14 may be disposed on a straight line, or may also be disposed by shifting one another in the circumferential direction as shown in Fig. 9. Further, spaces between the distal side perfusion ports 13 or between the proximal side perfusion ports 14 may be uniform, or may also be changed such that the spaces are made gradually smaller toward the distal side of the catheter. Each of the perfusion ports 13 and 14 preferably has a circular shape in view of easy processing. However, each of the perfusion ports 13 and 14 may be formed into an oval shape or other shapes depending on the circumstances.

The perfusion lumen 19 which connects the perfusion ports 13 and 14 to each other is formed by the balloon inside tube 20 which passes through the inner lumen of the balloon 5. The perfusion lumen 19 may be formed as a single lumen together with the guide wire lumen 10 as shown in Fig. 7, or may also be formed as a different lumen from the guide wire lumen 10 of the balloon inside tube 20 as shown in Fig. 8.

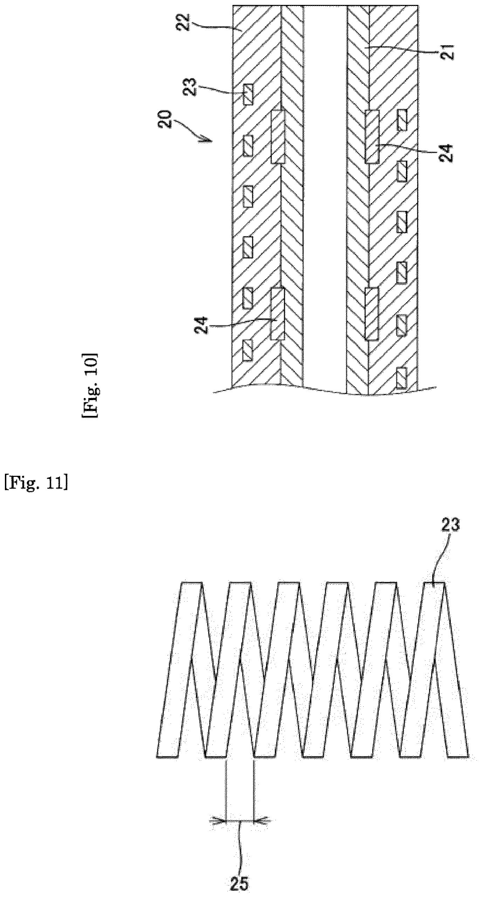

As shown in Fig. 10, the balloon inside tube 20 includes an inner layer 21, an outer layer 22, and a support body 23. A radiopaque marker 24 can be provided in the balloon inside tube 20 as necessary.

In the present invention, the inner layer 21 indicates a layer that forms the inner lumen formed in the balloon inside tube 20, and the outer layer 22 indicates a layer that covers the outer periphery of the inner layer 21.

Further, in the present invention, the balloon inside tube 20 is formed by heat-welding the outer layer 22 to the inner layer 21 and the support body 23 at a temperature that is equal to or higher than the melting point of a thermoplastic polymer that forms the outer layer 22 as well as lower than the melting point of a thermoplastic polymer that forms the inner layer 21. Therefore, sleeves of the balloon 5 can be firmly welded to the balloon inside tube 20. Further, even when a guide wire makes contact with the inner layer 21 such as a case where the guide wire is inserted into or removed from the inner lumen of the balloon inside tube 20, delamination of the inner layer 21 is not likely to occur.

The inner layer 21 of the balloon inside tube 20 is only required to be formed of a thermoplastic polymer that can be heat-welded to the balloon 5 and the outer layer 22 of the balloon inside tube 20 as well as has a higher melting point than a thermoplastic polymer that forms the outer layer 22. Since the inner layer 21 is formed of a thermoplastic polymer that can be heat-welded to the outer layer 22 of the balloon inside tube 20 and is the same kind of thermoplastic polymer as the material of the outer layer 22, the inner layer 21 and the outer layer 22 are not easily delaminated from each other.

Further, the thermoplastic polymer forming the inner layer 21 is heat-welded to a distal side sleeve 7 and can be heat-welded to a proximal side sleeve 8 of the balloon 5. Therefore, when the balloon 5 and the balloon inside tube 20 are heat-welded to each other, the distal side sleeve 7 and the proximal side sleeve 8 to be heat-welded can be integrally welded to the balloon inside tube 20. Therefore, the inner layer 21 and the outer layer 22 of the balloon inside tube 20 are not easily delaminated from each other at a heat-welded portion.

As the thermoplastic polymer that forms the inner layer 21, polyolefin, a polyolefin elastomer, polyester, a polyester elastomer, polyamide, a polyamide elastomer, polyurethane, a polyurethane elastomer, and the like can be used. When adequate flexibility, strength, and processability are taken into consideration, polyamide or a polyamide elastomer is preferably used. In particular, in view of easy forming of the balloon inside tube 20, a polyamide or a polyamide elastomer having a Shore D hardness of 63D or more is more preferably used. Since a polyamide or a polyamide elastomer having a Shore D hardness of 63D to 74D is generally used as the balloon 5 of a balloon catheter for PTCA, a polyamide or a polyamide elastomer having a Shore D hardness of 63D to 74D is further more preferably used as the inner layer 21 because the interfaces between the inner layer 21, the outer layer 22 and the balloon 5 can be more firmly welded when the balloon and the balloon inside tube 20 are heat-welded to each other.

When the inner layer 21 has a multi-layer structure, the highest Shore hardness among the Shore hardnesses of a plurality of thermoplastic polymers is determined as the Shore hardness of the inner layer 21.

"Shore D hardness" described in the present invention indicates a typical value for a particular material and may be different from a Shore D hardness measured by a general measurement method ISO868 (for example, in the case of "PEBAX^{®} 7033" manufactured by Arkema Inc. which is a polyamide elastomer, although a measured value of the Shore D hardness thereof is 69D, the Shore D hardness is determined as 70D in the present invention).

The thickness of the inner layer 21 may be appropriately determined depending on the intended use of the balloon catheter, and is only required to be in the range of 0.005 to 0.050 mm.

The outer layer 22 of the balloon inside tube 20 is only required to be formed of a thermoplastic polymer that can be heat-welded to the balloon 5 as well as has a lower melting point than the inner layer 21 of the balloon inside tube 20. When the outer layer 22 of the balloon inside tube 20 is formed of a material that can be heat-welded to the balloon 5, bonding with adhesive, which results in a rigid junction, is not necessary to be performed. Therefore, it is possible for the balloon catheter A to have pliability that is required for a catheter.

As the thermoplastic polymer that forms the outer layer 22, polyolefin, a polyolefin elastomer, polyester, a polyester elastomer, polyamide, a polyamide elastomer, polyurethane, a polyurethane elastomer, and the like can be used. When adequate flexibility, strength, and processability are taken into consideration, polyamide or a polyamide elastomer is preferably used. In order to improve the pliability of the balloon inside tube 20, a polyamide elastomer having a Shore D hardness of 55D or less is more preferably used. Further, in view of more adequate flexibility of the balloon catheter, a polyamide elastomer having a Shore D hardness of 40D to 55D is further more preferably used.

When the outer layer 22 has a multi-layer structure, the lowest Shore hardness among the Shore hardnesses of a plurality of thermoplastic polymers is determined as the Shore hardness of the outer layer 22.

In the present invention, the melting point of the thermoplastic polymer that forms the inner layer 21 is preferably higher than the melting point of the thermoplastic polymer that forms the outer layer 22 by 5 to 50°C, and more preferably by 8 to 25°C. When the difference between the melting point of the thermoplastic polymer forming the inner layer 21 and the melting point of the thermoplastic polymer forming the outer layer 22 is less than 5°C, the core member tends to become difficult to pull out. Further, when the difference exceeds 30°C, there is a tendency for the thermoplastic polymer forming the inner layer 21 and the thermoplastic polymer forming the outer layer 22 to become difficult to weld to each other, thereby lowering the adhesiveness between the inner layer 21 and the outer layer 22.

When the inner layer 21 has a multi-layer structure, the above-described melting point is directed to the melting point of a thermoplastic polymer forming a layer that has contact with the outer layer 22. Similarly, when the outer layer 22 has a multi-layer structure, the above-described melting point is directed to the melting point of a thermoplastic polymer forming a layer that has contact with the inner layer 21.

The thickness of the outer layer 22 may be appropriately determined depending on the intended use of the balloon catheter. In the case of a perfusion balloon catheter, the thickness of the outer layer 22 is only required to be in the range of 0.010 to 0.100.

The form of the support body 23 of the balloon inside tube 20 is not limited as long as the support body 23 achieves improvement of the strength or the like of the balloon inside tube 20. Metal such as superelastic alloy and stainless steel, and a high rigidity resin material such as polyimide, polyamide-imide, and polyetheretherketone can be used as the support body 23. When the support body 23 is disposed so as to be embedded in the outer layer 22 and extend the entire length of the balloon inside tube 20, it is possible to improve the pressing performance of the balloon catheter. In the present invention, since an outer tube 28 for forming the outer layer 22 shrinks while being melted by heat-welding, the support body 23 exists in an embedded state in the outer layer 22.

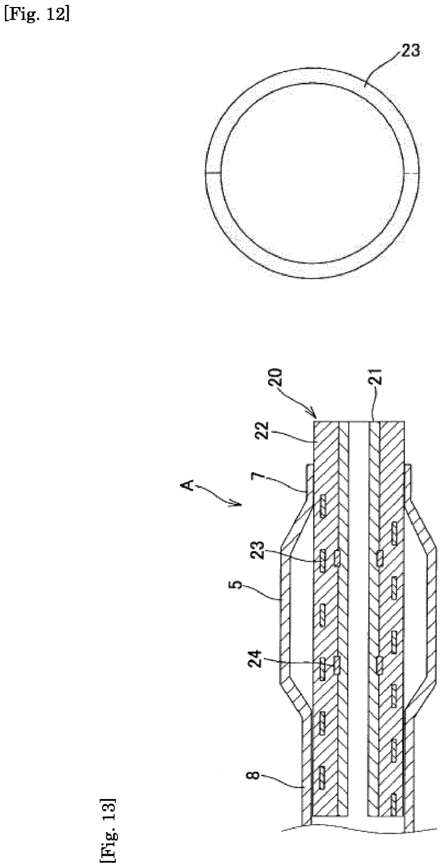

In the present invention, in order to improve the buckling strength in the radial direction, the support body 23 may be disposed so as to extend over the entire circumference of the balloon inside tube 20. In this case, the support body 23 preferably has a coil structure or a braid structure. For example, when the support body 23 is formed into a coil shape as shown in Figs. 11 and 12, it is possible to improve the buckling strength in the radial direction of the balloon-provided part and also impart pliability to the balloon inside tube 20. In this case, a wire-like support body 23 may be wound around an inner tube 26 for forming the inner layer 21 when forming the balloon inside tube 20, or the wire may first be formed into a coil shape and then disposed on the inner tube 26. Further, when the support body 23 is formed to have a braid structure, it is possible to improve the buckling strength in the radial direction of the balloon-provided part and the pressing performance for transmitting force applied to the proximal side of the catheter toward the distal side thereof.

The shape of the wire that forms the support body 23 is not particularly limited, and the cross section of the wire can be formed into a rectangular shape or a round shape. In the case of a rectangular wire, improvement of the pressing performance in the axial direction can be expected. In the case of a round wire, improvement of the pliability can be expected. The cross-sectional area of the wire can be freely designed in view of the buckling strength, the pressing performance, the pliability and the like.

As shown in Fig. 11, in the support body 23 having a coil shape, the length of a pitch 25 between windings of the coil may be any value as long as it is larger than zero. The shorter the length of the pitch 25 is, the more the buckling strength is improved. When the length of the pitch 25 is zero, since the outer tube 28 for forming the outer layer 22 does not reach the surface of the inner tube 26 when forming the balloon inside tube 20 by heat-welding, the balloon cannot be formed.

In addition, in order to confirm the position of the balloon 5 inside the body, the radiopaque marker(s) 24 may be disposed on the balloon inside tube 20. The support body 23 of the balloon inside tube 20 may have radiopaque property, or the radiopaque marker(s) 24 may be provided other than the support body 23. Especially when the outer surface of the radiopaque marker(s) 24 is not exposed on the outer surface of the balloon inside tube 20, the radiopaque marker(s) 24 does not hurt the balloon 5. As a result, the probability of occurrence of a failure such as balloon rupture can be reduced. When the radiopaque marker(s) 24 is located at a more inner position in the radial direction than the support body 23 of the balloon inside tube 20, it is possible to reduce deviation of the support body 23 in the axial direction when forming the balloon inside tube 20.

In view of high efficiency, the balloon inside tube 20 having the above structure is produced in accordance with the steps of
inserting a core member into an inner tube for forming the inner layer 21 of the balloon inside tube 20, the inner layer 21 being formed of a thermoplastic polymer that can be heat-welded to the balloon 5;
disposing the support body 23 on the outer surface of the inner tube;
disposing an outer tube for forming the outer layer 22 of the balloon inside tube 20, the outer layer 22 being formed of a thermoplastic polymer that can be heat-welded to the balloon 5, so as to cover the support body 23;
disposing a heat-shrinkable tube so as to cover the outside of the outer tube;
performing heat-welding of the outer layer 22 to the inner layer 21 and the support body 23 at a temperature equal to or higher than the melting point of the thermoplastic polymer forming the outer layer 22 as well as lower than the melting point of the thermoplastic polymer forming the inner layer 21;
removing the heat-shrinkable tube;
removing the core member to obtain the balloon inside tube 20; and
heat-welding the balloon 5 at a distal side thereof to a distal part of the balloon inside tube 26 at a temperature equal to or higher than the melting point of the thermoplastic polymer forming the inner layer 21 of the balloon inside tube 26, wherein the thermoplastic polymer forming the inner layer 21 has a higher melting point than the thermoplastic polymer forming the outer layer 22.

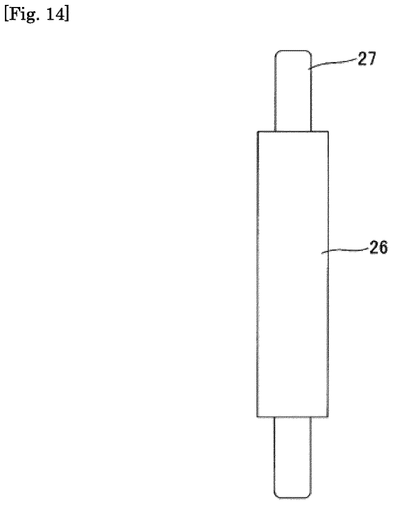

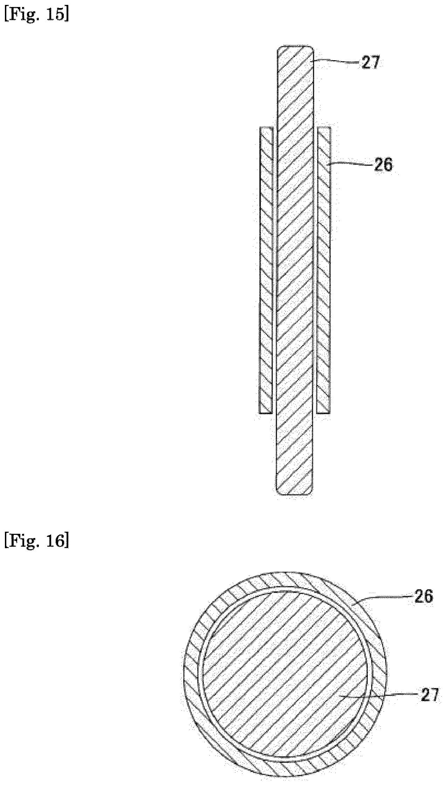

Specifically, as shown in Figs. 14 to 16, the lumen forming core member 27 is first inserted into the inner tube 26 for forming the inner layer 21 of the balloon inside tube 20.

The inner tube 26 may have either a single-layer structure or a multi-layer structure as long as at least it can form the inner layer 21. When the inner tube 26 has a multi-layer structure, the inner layer 21 may be formed by the most inner layer, and the other layers may be formed of any thermoplastic polymer as long as it can be heat-welded to the balloon 5. In order to improve "pressing performance" for transmitting force applied to the proximal side of the catheter to the distal side thereof, a rigid material can be used. However, in order for the catheter to pass through a curved blood vessel, a pliable material can also be used.

Further, in order to more easily remove the lumen forming core member 27 by forming the surface of the inner lumen of the balloon inside tube 20 into a rough surface when forming the balloon inside tube 20, the inner layer 21 may contain fine particles such as pigment. The fine particles used in this case may be any particles as long as the diameter thereof is 0.1 mm or less. Examples of the fine particles include, in addition to pigment, inorganic filler such as titanium oxide, calcium carbonate, alumina, and silica, and resin filler such as acrylic resin, silicone resin, and carbon.

The fine particles may be mixed with the thermoplastic polymer which is the material of the inner tube 26 when producing the inner tube 26.

The size and the length of the lumen forming core member 27 may be appropriately determined depending on the purpose and intended use of the balloon catheter.

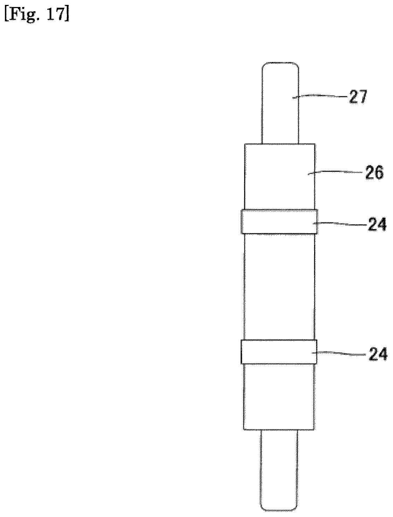

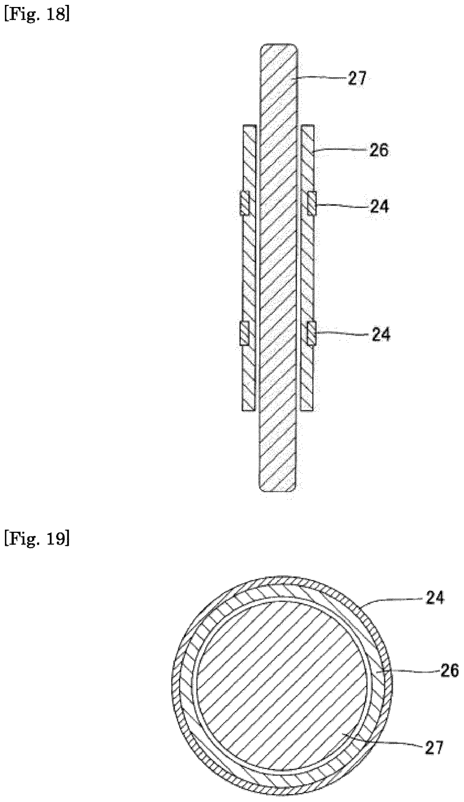

If necessary, the radiopaque marker(s) 24 is attached to the outside of the inner tube 26 as shown in Figs. 17 to 19.

In this case, the radiopaque marker(s) 24 is preferably swaged onto the outer surface of the inner tube 26. Swaging the radiopaque marker(s) 24 indicates, for example, a state where the radiopaque marker(s) 24 is embedded in the inner layer 21 from the surface toward the inside thereof as shown in Fig. 10. Swaging means is not particularly limited.

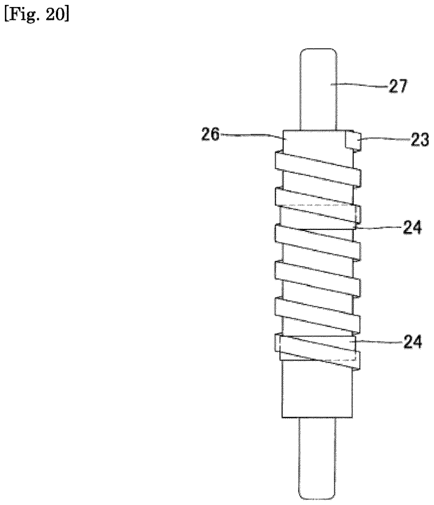

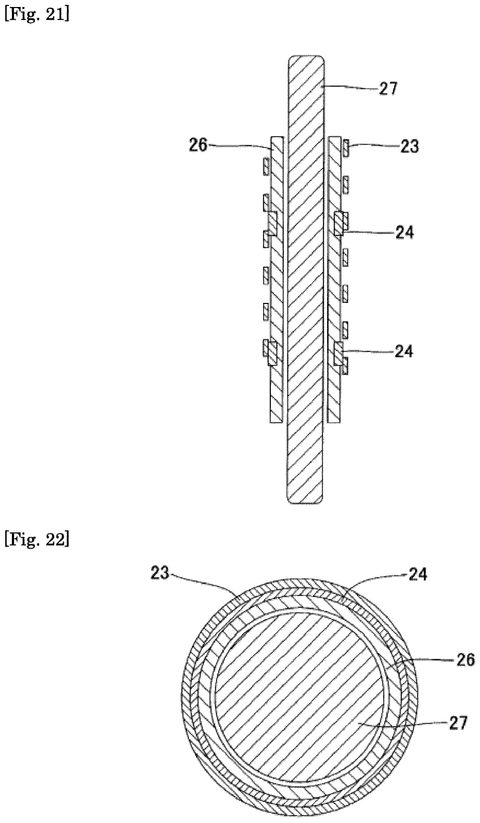

Then, the support body 23 is disposed on the outer surface of the inner tube 26 (when the radiopaque marker(s) 24 is provided, the outer surface(s) of the radiopaque marker(s) 24) as shown in Figs. 20 to 22. A method for disposing the support body 23 is not particularly limited. For example, when the support body 23 is formed of a rectangular wire and has a coil shape, the support body 23 may be previously formed into a coil shape, and the inner tube 26 or the like may be inserted into the support body 23 having a coil shape. Alternatively, the support body 23 may be wound around the outer surface of the inner tube 26 into a coil shape by a conventional method. Further, the catheter inside tube to be obtained tends to become harder when the support body 23 is disposed closer to the surface of the inner tube 26. Therefore, by adjusting the position of the support body 23, desired flexibility of the catheter inside tube can be adjusted. The disposing position of the support body 23 is not particularly limited. The support body 23 may be disposed only within the inner lumen of the balloon 5 when the balloon 5 is connected to the balloon inside tube 20, may extend to the sleeves of the balloon 5, or may extend beyond the sleeves.

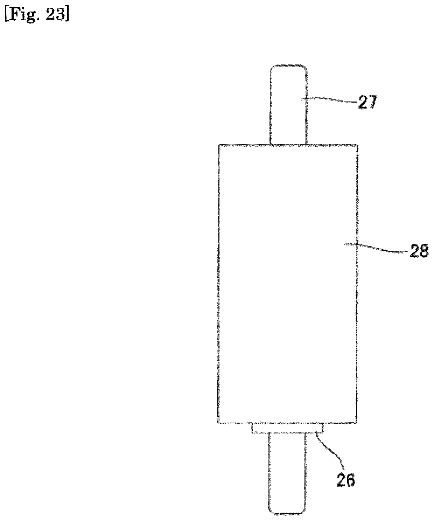

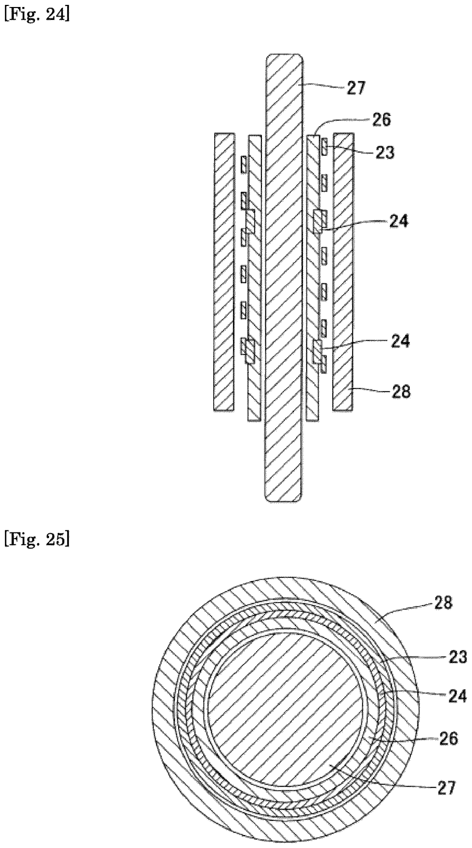

Then, the outer tube 28 for forming the outer layer 22 of the balloon inside tube 20 is disposed on the outside of the support body 23 as shown in Figs. 23 to 25.

The outer tube 28 may have either a single-layer structure or multi-layer structure as long as it is formed of a thermoplastic polymer that can be heat-welded to the balloon 5 as well as has a lower melting point than the inner layer 21 of the balloon inside tube 20. In order to improve "pressing performance", a rigid material can be used. However, in order for the catheter to pass through a curved blood vessel, a pliable material can also be used.

Further, the outer tube 28 may be disposed so as to approximately entirely cover the inner tube 26.

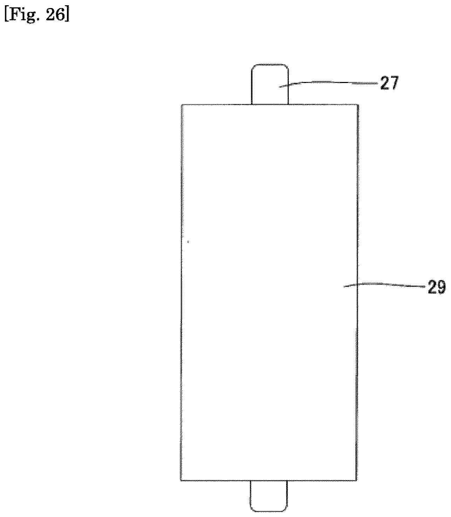

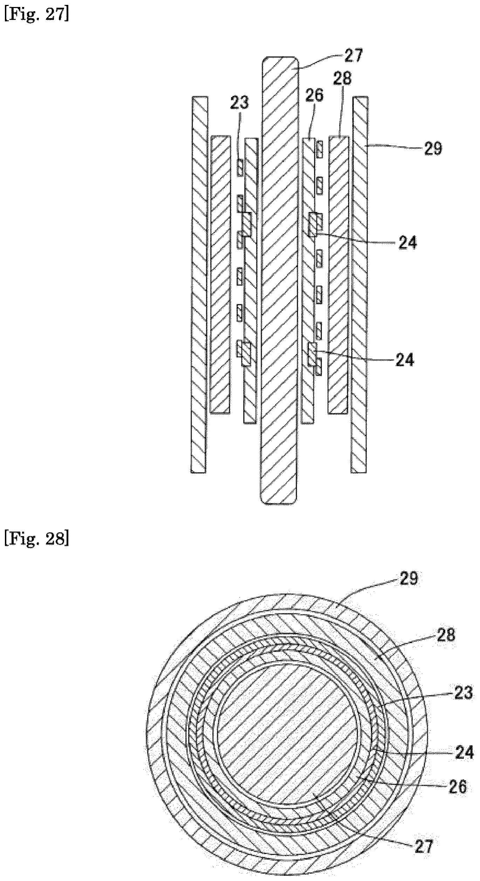

Then, the heat-shrinkable tube 29 is disposed so as to cover the outside of the outer tube 28 as shown in Figs. 26 to 28. The heat-shrinkable tube 29 has a property of shrinking by heat. The material of the heat-shrinkable tube 29 is not particularly limited as long as it is used in the art.

Then, the heat-shrinkable tube 29 is heated at a temperature that is equal to or higher than the melting point of the thermoplastic polymer that forms the outer layer 22 as well as lower than the melting point of the thermoplastic polymer that forms the inner layer 21. In this manner, the outer tube 28 which is disposed in the inner lumen of the heat-shrinkable tube 29 is melted and thereby heat-welded to the surfaces of the support body 23 and the inner tube 26.

In this regard, "melting point" referred to in the present invention indicates a melting point measured by an ASTM D3418 method.

As described above, by performing the heat-welding at a temperature that is equal to or higher than the melting point of the thermoplastic polymer that forms the outer layer 22 as well as lower than the melting point of the inner layer 21, the outer tube 28 is melted and thereby adheres to the outer surface of the inner tube 26 (and the surface(s) of the radiopaque marker(s) 24) while the support body 23 is being embedded into the melted outer tube 28 as shown in Fig. 10. The outer layer 22 formed in this manner is integrated with the inner layer 21 and the support body 23, thereby making it possible to form the balloon inside tube 20 having no defect such as a gap inside thereof.

Further, in the present invention, by performing the heat-welding under the temperature condition described above, the inner tube 26 is not melted and is not therefore stuck to the lumen forming core member 27. Therefore, it is easy to remove the lumen forming core member 27 after forming the balloon inside tube 20.

A method of the heat-welding is not particularly limited, and a hot air welding machine and an oven can be used. The heat-welding method may be any method as long as it can bring the temperature of an object into a temperature approximately equal to the above-described one by exposing the object under the atmosphere at a temperature of 160 to 168°C, for example.

Then, the heat-shrinkable tube 29 can be removed from the inner tube 26 to produce the balloon inside tube 20. The heat-shrinkable tube 29 is preferably removed after the outer tube 28 is cooled to transition from the melted state.

When the balloon inside tube 20 has a biaxial structure, the balloon inside tube 20 may be produced in such a manner that the inner tube 26 having a biaxial structure is previously produced, two lumen forming core members 27 are inserted into respective two lumens of the inner tube 26, and the radiopaque marker(s) 24, the support body 23, and the outer tube 28 are disposed on the outside of the inner tube 26. Alternatively, the balloon inside tube 20 may be produced in such a manner that two inner tubes 26 each of which has the lumen forming core member 27 inserted thereinto are disposed in parallel to each other, and the radiopaque marker(s) 24, the support body 23, and the outer tube 28 are disposed on the outside of the inner tubes 26.

In order to reduce frictional resistance between the balloon inside tube 20 and the guide wire 18 which passes through the inside of the balloon inside tube 20 when performing PTCA, lubricant coating may be applied to the inner surface of the inner layer 21 of the balloon inside tube 20 after forming the balloon inside tube 20. Examples of the lubricant coating agent include a silicone coating agent such as silicone and polydimethylsiloxane, and an acrylic coating agent such as butyl (meth) acrylate, methyl (meth) acrylate, ethyl (meth) acrylate, propyl (meth) acrylate, octyl (meth) acrylate, and 2, 2, 2-trifluoroethyl methacrylate. The lubricant coating agent may be applied to the inner surface of the inner layer 21 by a known method.

The balloon 5 is heat-welded to the outer surface of the balloon inside tube 20 formed as described above. The balloon 5 is only required to be formed of a thermoplastic polymer that can be heat-welded to the outer layer 22 of the balloon inside tube 20, and any other limitations are not particularly placed on the material of the balloon 5. Polyolefin, a polyolefin elastomer, polyester, a polyester elastomer, polyamide, a polyamide elastomer, polyurethane, a polyurethane elastomer and the like can be used as the material of the balloon 5. When adequate flexibility, strength, and processability are taken into consideration, polyamide or a polyamide elastomer is preferably used.

The entire length of the balloon 5 including the distal side sleeve 7 and the proximal side sleeve 8 may be appropriately determined depending on the intended use of the balloon catheter. In the case of a perfusion balloon catheter, the length is only required to be in the range of 10.0 to 40.0 mm. In the case of a perfusion balloon catheter, the diameter of a straight tube portion of the balloon 5 is also only required to be in the range of 2.0 to 4.5 mm. Although the thickness of the balloon 5 is also not particularly limited, the thicknesses of both of the straight tube portion and the sleeves are preferably in the range of 0.015 to 0.040 mm. Further, as shown in Fig. 13, the end of the distal side sleeve 7 of the balloon 5 may be located at a more proximal position than the distal end of the balloon inside tube 20.

In the present invention, the distal side sleeve 7 which is positioned at the distal side of the distal part of the balloon 5 and the distal part of the balloon inside tube 20 are heat-welded. By performing the heat-welding at a temperature equal to or higher than the melting point of the thermoplastic polymer that forms the inner layer 21, the distal side sleeve 7, and the outer layer 22 and the inner layer 21 of the balloon inside tube 20 are integrated with each other, thereby making it possible to further suppress the delamination of the inner layer 21 of the balloon inside tube 20.

In addition, the proximal side sleeve 8 which is positioned at the proximal side of the proximal part of the balloon 5 and the proximal part of the balloon inside tube 20 are also heat-welded in the same manner as in the distal part thereof. As a result, the balloon 5, and the outer layer 22 and the inner layer 21 of the balloon inside tube 20 are integrated with each other in the welded portions, and the delamination of the inner layer 21 is thereby further suppressed.

For example, a core member is previously inserted between a part of the proximal side sleeve 8 and the balloon inside tube 20, and the other part of the proximal side sleeve 8, the other part not having contact with the core member, is heat-welded to the surface of the balloon inside tube 20. Thereafter, the core member is pulled out to thereby form a lumen. As a result, it is possible to allow the inner lumen of the balloon 5 and the inflation lumen 12 of the distal side shaft 4 to communicate with each other through the formed lumen.

Further, as shown in Figs. 7 and 8, a part of the proximal side sleeve 8 of the balloon 5 may also be brought to butt against the end of the distal side shaft 4 without having contact with the balloon inside tube 20. In this case, the end of the balloon inside tube 20 butts against the end of the inside tube of the distal side shaft 4. The upper part of the inner lumen of the balloon 5 communicates with the inflation lumen 12 of the distal side shaft 4. The lower part of the inner lumen of the balloon 5 is closed by the end of the distal side shaft 4. The other circumference of the proximal side sleeve 8 may be heat-welded on the surface of the balloon inside tube 20.

Although not shown in the drawings, the end of the distal side shaft 4 may be inserted between the proximal side sleeve 8 and the balloon inside tube 20, and the surface of the distal side shaft 4 and the proximal side sleeve 8 may be heat-welded to each other in the same manner as in a common balloon catheter.

The distal end part including the balloon 5 which is the characterizing portion of the present invention is formed in the above-described manner.

In the present invention, the configurations other than the configuration of the distal end part, that is, the specific configurations of the distal side shaft 4, the proximal side shaft 6, and the hub 9 as shown in Fig. 1 may be the same as those in a common balloon catheter. The distal side shaft 4 may be formed by connecting a plurality of shafts having different cross-sectional shapes in the radial direction. A method for connecting the distal side shaft 4, the proximal side shaft 6, and the hub 9 to each other may also be the same as that in a common balloon catheter.

### Examples

Hereinbelow, specific examples according to the present invention will be described in detail. However, the present invention is not limited to the following examples.

### (Example 1)

A balloon catheter that includes a balloon inside tube 20 including an inner layer 21 which is formed of a polyamide elastomer (Shore D hardness: 63D, melting point: 169°C), an outer layer 22 which is formed of a polyamide elastomer (Shore D hardness: 40D, melting point: 160°C), and a support body 23 which is formed of a metal wire (SUS304), and a balloon 5 which is formed of a polyamide elastomer (Shore D hardness: 70D, melting point: 172°C) was produced according to the following procedure.

As shown in Figs. 14 to 16, a lumen forming core member 27 (SUS304, diameter: 0.87 mm) the surface of which is coated with parylene was inserted into an inner tube 26 (length: 6.0 cm, inner diameter: 0.88 mm, thickness: 0.035 mm) for forming the balloon inside tube 20, the inner tube 26 being formed of a polyamide elastomer (Shore D hardness: 63D, melting point: 169°C). Further, as shown in Figs. 17 to 19, radiopaque markers 24 (made of a platinum-iridium alloy) each of which is formed into a band of 1mm width were swaged onto the outer surface of the inner tube 26.

Then, as shown in Figs. 20 to 22, a coil-shaped support body 23 (diameter: 1.02 mm, pitch width: 0.15 mm) which is formed of a rectangular wire was disposed on the outer surface of the inner tube 26 and the outer surfaces of the radiopaque markers 24. The length of the coil-shaped support body 23 in the axial direction was made to be the same as the length from the distal end of a distal side sleeve to the proximal end of a proximal side sleeve of the balloon to be heat-welded to the balloon inside tube later.

Then, as shown in Figs. 23 to 25, an outer tube 28 (length: 5.5 cm) which is formed of two kinds of polyamide elastomers (Shore D hardness: 40D, thickness: 0.040 mm, melting point: 160°C in an outer layer; Shore D hardness: 55D, thickness: 0.010 mm, melting point: 159°C in an inner layer) was disposed so as to cover the outside of the coil-shaped support body 23.

Then, as shown in Figs. 26 to 28, a heat-shrinkable tube 29 (made of polyolefin, shrinkage temperature: 120°C or higher, shrinkage rate: 40% or higher, inner diameter before shrinking: approximately 1.6 mm) was disposed on the outside of the outer tube 28, put in an oven maintained at a constant temperature of 165°C, and taken out from the oven 15 minutes later. Then, the heat-shrinkable tube 29 and the lumen forming core member 27 were removed therefrom to form the balloon inside tube 20 having the structure as shown in Fig. 10 (length after being cut: 5.0 cm, inner diameter: 0.87 mm, outer diameter: 1.10 mm).

Apart from the above, a tube-shaped parison was produced by extrusion molding using a polyamide elastomer (Shore D hardness: 70D). Then, blow molding was performed using the parison to produce the balloon 5 which includes a straight tube portion having an outer diameter of 3 mm, a length of 2 cm, and a thickness of 0.02 mm. The distal side sleeve 7 which was provided in the straight tube portion has an outer diameter of 1. 25 mm, an inner diameter of 1.15 mm, and a length of 5 mm. The proximal side sleeve 8 which was provided in the straight tube portion has an outer diameter of 1. 30 mm, an inner diameter of 1.20 mm, and a length of 2 mm. The entire length of the balloon 5 was 2.75 cm.

As shown in Fig. 13, the balloon 5 was disposed on the balloon inside tube 20 so that the distal side part of the balloon inside tube 20 overlaps with the distal side sleeve 7 and the proximal side part of the balloon inside tube 20 overlaps with the proximal side sleeve 8. In addition, the balloon 5 was disposed so that the end part of the balloon inside tube 20 protrudes by 5 mm from the end of the distal side sleeve 7, and the thus protruding part of the balloon inside tube 20 was cut to form the distal end of the balloon inside tube 20.

Then, the distal side part of the balloon inside tube 20 and the distal side sleeve 7 were heat-welded to each other at a temperature that is equal to or higher than the melting point of the material of the inner layer 21 of the balloon inside tube 20 (180 to 220°C).

Then, the distal end of a two-hole tube (length: 3.0 cm, outer diameter: 1.13 mm, maximum diameter of guide wire lumen 10: 0.86 mm, maximum diameter of inflation lumen: 0.58 mm) that has the same cross-sectional configuration as that shown in Fig. 4 and is made of a polyamide elastomer was brought to butt against the proximal end of the balloon inside tube 20, and the proximal side part of the balloon inside tube 20, the distal part of the two-hole tube, and the proximal side sleeve 8 were heat-welded to each other. Specifically, as shown in Fig. 7, the end of the proximal side sleeve 8 of the balloon 5 and the end of the two-hole tube (the distal side shaft 4) were heat-welded to each other (at 180 to 220°C) so that the inflation lumen 12 of the two-hole tube to be the distal side shaft 4 communicates with the inside of the balloon 5. In addition, the end of the balloon inside tube 20 and the end of the two-hole tube were heat-welded to each other (at 180 to 220°C) so that the guide wire lumen 10 of the two-hole tube communicates with the balloon inside tube 20.

Then, the proximal part of the two-hole tube was connected to a shaft that has a double-tube structure including an outer tube and an inner tube. Specifically, the distal part of a distal end side outer tube (length: 24.0 cm, inner diameter: 0.94 mm, outer diameter: 1.11 mm) which is formed of a polyamide elastomer and the distal part of a distal end side inner tube (length: 24.0 cm, inner diameter: 0.43 mm, outer diameter: 0.58 mm) which is formed of a polyamide elastomer and polyethylene were heat-welded to the proximal part of the two-hole tube to thereby connect the two kinds of distal side shafts to each other.

Then, as a proximal side shaft to be connected to the distal side shaft, a shaft that includes a metal tube (length: 110 cm, inner diameter: 0.45 mm, outer diameter: 0.63 mm) which is formed of SUS304 coated with polytetrafluoroethylene and an intermediate tube (length: 10 cm, inner diameter: 0.85 mm, outer diameter: 1.00 mm) which is connected to the distal side of the metal tube and made of a polyamide elastomer was used. In order to improve continuity of rigidity, a metal wire which is made of SUS304 is disposed from the intermediate tube through the distal side shaft, and the distal side shaft 4 and the proximal side shaft 6 were connected to each other so that a proximal side guide wire port is formed on the junction between the distal side shaft 4 and the proximal side shaft 6 as shown in Fig. 1. The metal wire (length: 25.0 cm, maximum outer diameter: 0.35 mm) was disposed in such a manner that the distal end of the metal wire is positioned 15 cm away from the proximal side guide wire port at the distal side thereof.

A hub 9 is attached to the proximal end of the proximal side shaft 6 so that a device for inflating the balloon 5 can be connected to the hub 9, thereby completing the production of the balloon catheter.

As shown in Fig. 7, sixteen proximal side perfusion ports 14 were formed on the wall of the two-hole tube (the distal side shaft 4), the wall facing the guide wire lumen 10 (which also serves as the perfusion lumen 19), so as to be aligned on a straight line at regular intervals. Further, eight distal side perfusion ports 13 in total were formed on the wall of the distal side sleeve 7 of the balloon 5 in such a manner that four sets of distal side perfusion ports 13, each set having two distal side perfusion ports 13 shifted in phase by 180° from each other in the circumferential direction, are positioned at regular intervals as well as shifted in phase by 90° from each other in the axial direction. In this manner, the perfusion balloon catheter was produced.

### (Example 2)

A balloon catheter that includes a balloon inside tube 20 including an inner layer 21 which is formed of a polyamide elastomer (Shore D hardness: 72D, melting point: 174°C), an outer layer 22 which is formed of a polyamide elastomer (Shore D hardness: 55D, melting point: 159°C) and a support body 23 which is formed of a metal wire (SUS304), and a balloon 5 which is formed of a polyamide elastomer (Shore D hardness: 72D, melting point: 174°C) was produced according to the following procedure.

The balloon catheter was produced in the same manner as in Example 1 excepting that polyamide elastomers (Shore D hardness: 55D in an outer layer, Shore D hardness: 72D in an inner layer) were used as the materials of an inner tube 26, a polyamide elastomer (Shore D hardness: 55D) was used as the material of an outer tube 28, a coil-shaped rectangular wire was used as the support body 23, and a polyamide elastomer (Shore D hardness: 72D) was used as the material of the balloon 5. The thickness of the outer layer of the inner tube 26 is 0.025 mm, the thickness of the inner layer of the inner tube 26 is 0.010 mm, and the thickness of the outer tube 28 is 0.50 mm.

### (Example 3)

A balloon catheter that includes a balloon inside tube 20 including an inner layer 21 which is formed of a polyamide (Shore D hardness: 74D, melting point: 174 to 178°C), an outer layer 22 which is formed of a polyamide elastomer (Shore D hardness: 55D, melting point: 159°C) and a support body 23 which is formed of a metal wire (SUS304), and a balloon 5 which is formed of a polyamide elastomer (Shore D hardness: 63D, melting point: 169°C) was produced according to the following procedure.

The balloon catheter was produced in the same manner as in Example 1 excepting that a polyamide (Shore D hardness: 74D, melting point: 174 to 178°C) was used as the material of the inner tube 26, a polyamide elastomer (Shore D hardness: 55D, melting point: 159°C) was used as the material of the outer tube 28, a coil-shaped round wire was used as the support body 23, and a polyamide elastomer (Shore D hardness: 63D, melting point: 169°C ) was used as the material of the balloon 5.

### (Comparative Example 1)

A balloon catheter that includes a balloon inside tube 20 including an inner layer 21 which is formed of a polytetrafluoroethylene (Polyflon^{®} PTFE F207, surface-treated, manufactured by Daikin Industries, Ltd., melting point: 380°C), an outer layer 22 which is formed of a polyamide elastomer (Shore D hardness: 70D, melting point: 172°C), and a support body 23 which is formed of a metal wire (SUS304), and a balloon 5 which is formed of a polyamide elastomer (Shore D hardness: 70D, melting point: 172°C) was produced according to the following procedure.

The balloon catheter was produced in the same manner as in Example 1 excepting that the above polytetrafluoroethylene was used as the material of the inner tube 26, a polyamide elastomer (Shore D hardness: 70D, melting point: 172°C) was used as the material of the outer tube 28, a coil-shaped rectangular wire was used as the support body 23, a polyamide elastomer (Shore D hardness: 70D, melting point: 172°C) was used as the material of the balloon 5, and a heat-welding temperature at the time of forming the balloon inside tube 20 was 180°C.

### (Comparative Example 2)

A balloon catheter that includes a balloon inside tube 20 including an inner layer 21 which is formed of a tetrafluoroethylene/perfluoroalkylvinyl ether copolymer (Neoflon^{®} PFAAP210, surface-treated, manufactured by Daikin Industries, Ltd., melting point: 380°C), an outer layer 22 which is formed of a polyamide elastomer (Shore D hardness: 72D, melting point: 174°C), and a support body 23 which is formed of a metal wire (SUS304), and a balloon 5 which is formed of a polyamide elastomer (Shore D hardness: 72D, melting point: 174°C) was produced according to the following procedure.

The balloon catheter was produced in the same manner as in Example 1 excepting that the above tetrafluoroethylene/perfluoroalkylvinyl ether copolymer was used as the material of the inner tube 26, a polyamide elastomer (Shore D hardness: 72D, melting point: 174°C) was used as the material of the outer tube 28, a coil-shaped rectangular wire was used as the support body 23, a polyamide elastomer (Shore D hardness: 72D, melting point: 174°C) was used as the material of the balloon 5, and a heat-welding temperature at the time of forming the balloon inside tube 20 was 185°C.

### (Comparative Example 3)

A balloon inside tube 20 that includes an inner layer 21 and an outer layer 22 each of which is formed of a polyamide (Shore D hardness: 55D, melting point: 159°C), and a support body 23 which is formed of a metal wire (SUS304) was produced according to the following procedure.

A lumen forming core member 27 (SUS304) the surface of which is coated with parylene was inserted into an inner tube 26 for forming the balloon inside tube 20, the inner tube 26 being formed of a polyamide elastomer (Shore D hardness: 55D, melting point 159°C). Radiopaque markers 24 (platinum-iridium alloy) were swaged onto the outer surface of the inner tube 26. Further, the coil-shaped support body 23 which is formed of a round wire was disposed on the outer surface of the inner tube 26 and the outer surfaces of the radiopaque markers 24. The length of the coil-shaped support body 23 in the axial direction was made to be the same as the length from the distal end of a distal side sleeve 7 to the proximal end of a proximal side sleeve 8. An outer tube 28 which is formed of a polyamide elastomer (Shore D hardness: 55D, melting point 159°C) was disposed so as to cover the outside of the coil-shaped support body 23. Further, a heat-shrinkable tube 29 (made of polyolefin, shrinkage temperature: 120°C or higher, shrinkage rate: 40% or higher, inner diameter before shrinking: approximately 1.6 mm) was disposed on the outside of the outer tube 28, put in an oven maintained at a constant temperature of 165°C, and taken out from the oven 15 minutes later. Then, the heat-shrinkable tube 29 was removed therefrom. However, the lumen forming core member 27 could not be pulled out therefrom.

### (Experiment Example)

In each of the balloon catheters produced in Examples 1 to 3 and Comparative Examples 1 and 2, a core member (diameter: 0.85 mm, material: SUS304) the end of which is not round-treated was continuously inserted into and removed from the guide wire lumen 10 (the inner lumen of the balloon inside tube 20) by approximately 3 cm while the balloon was being inflated by inserting water thereinto at 20 atm. In the balloon catheters of Examples 1 to 3, the inner layer 21 of the balloon inside tube 20 was not delaminated within 100 times of insertion/removal of the core member. However, in the balloon catheters obtained in Comparative Examples 1 and 2, delamination of the inner layer 21 of the balloon inside tube 20 was confirmed within 20 times of insertion/removal of the core member.

### Reference Signs List

- A, A': balloon catheter
- 1: distal side guide wire port
- 2: proximal side guide wire port
- 3: inflation port
- 4: distal side shaft
- 4X: distal side shaft distal end
- 5: balloon
- 6: proximal side shaft
- 7: distal side sleeve
- 8: proximal side sleeve
- 9: hub
- 10: guide wire lumen
- 11: inside tube
- 12: inflation lumen
- 13: distal side perfusion port
- 14: proximal side perfusion port
- 15: blood vessel
- 16: blood
- 17: flowing direction of blood
- 18: guide wire
- 19: perfusion lumen
- 20: balloon inside tube
- 21: inner layer of balloon inside tube
- 22: outer layer of balloon inside tube
- 23: support body of balloon inside tube
- 24: radiopaque marker
- 25: pitch between windings of support body
- 26: inner tube
- 27: lumen forming core member
- 28: outer tube
- 29: heat-shrinkable tube

## Claims

1. A balloon catheter (A; A') comprising:
a balloon (5); and
a balloon inside tube (26) passing through an inner lumen of the balloon (5), the balloon inside tube (26) including an inner layer (21), an outer layer (22), and a support body (23),
wherein the balloon (5), the inner layer (21), and the outer layer (22) are formed of thermoplastic polymers that can be heat-welded to each other, and
a thermoplastic polymer forming the inner layer (21) has a higher melting point than a thermoplastic polymer forming the outer layer (22);
**characterized in that**
the balloon (5) has a distal side sleeve (7) at a distal side thereof, and the distal side sleeve (7) is heat-welded to a distal part of the balloon inside tube (26) at a temperature equal to or higher than the melting point of the thermoplastic polymer forming the inner layer (21) of the balloon inside tube (26).

2. The balloon catheter (A; A') according to claim 1, wherein the balloon inside tube (26) is formed by heat-welding the outer layer (22) to the inner layer (21) and the support body (23) at a temperature that is equal to or higher than the melting point of the thermoplastic polymer forming the outer layer (22) as well as lower than the melting point of the thermoplastic polymer forming the inner layer (21).

3. The balloon catheter (A; A') according to claim 1 or 2, wherein the balloon (5) has a proximal side sleeve (8) at a proximal side thereof, and the proximal side sleeve (8) is heat-welded to a proximal part of the balloon inside tube (26) at a temperature equal to or higher than the melting point of the thermoplastic polymer forming the inner layer (21) of the balloon inside tube (26).

4. The balloon catheter (A; A') according to any one of claims 1 to 3, wherein each of the thermoplastic polymers is polyamide or a polyamide elastomer.

5. The balloon catheter (A; A') according to any one of claims 1 to 4, wherein the Shore D hardness of the inner layer (21) is in the range of 63D to 74D.

6. The balloon catheter (A; A') according to any one of claims 1 to 5, wherein the Shore D hardness of the outer layer (22) is in the range of 40D to 55D.

7. The balloon catheter (A; A') according to any one of claims 1 to 6, wherein the support body (23) is embedded in the outer layer (22), extends at least over a part of the balloon inside tube (26), the part being positioned within the inner lumen of the balloon, and extends throughout the entire circumferential direction of the balloon inside tube (26).

8. The balloon catheter (A; A') according to claim 7, wherein the support body (23) is a coil-shaped support body (23).

9. The balloon catheter (A; A') according to claim 8, wherein the coil-shaped support body (23) is formed of a rectangular wire.

10. The balloon catheter (A; A') according to claim 8, wherein the coil-shaped support body (23) is formed of a round wire.

11. The balloon catheter (A; A') according to any one of claims 1 to 10, further comprising a radiopaque marker (24) located within the balloon inside tube (26).

12. The balloon catheter (A; A') according to claim 11, wherein the radiopaque marker (24) is located at more inner position in the radial direction than the support body (23).

13. The balloon catheter (A; A') according to any one of claims 1 to 12, wherein the balloon inside tube (26) is connected to a distal end of a distal side shaft (4), a proximal side shaft (6) is connected to a proximal end of the distal side shaft (4), and a hub (9) is attached to a proximal part of the proximal side shaft (6).

14. The balloon catheter (A; A') according to any one of claims 1 to 13, wherein the balloon catheter (A; A') is a perfusion balloon catheter (A; A'), and an inner lumen of the balloon inside tube (26) forms a perfusion lumen.

15. A method for producing a balloon catheter (A; A') including a balloon, and a balloon inside tube (26) passing through an inner lumen of the balloon (5), the balloon inside tube (26) including an inner layer (21), an outer layer (22), and a support body (23), the method comprising the steps of:
inserting a core member (27) into an inner tube (26) for forming the inner layer (21) of the balloon inside tube (26), the inner tube (26) being formed of a thermoplastic polymer that can be heat-welded to the balloon (5);
disposing the support body (23) on an outer surface of the inner tube (26);
disposing an outer tube (28) for forming the outer layer (22) of the balloon inside tube (26), the outer tube (28) being formed of a thermoplastic polymer that can be heat-welded to the balloon (5), so as to cover the support body (23);
disposing a heat-shrinkable tube so as to cover the outside of the outer tube (28);
performing heat-welding of the outer layer (22) to the inner layer (21) and the support body (23) at a temperature that is equal to or higher than the melting point of the thermoplastic polymer forming the outer layer (22) as well as lower than the melting point of the thermoplastic polymer forming the inner layer (21);
removing the heat-shrinkable tube;
removing the core member (27) to obtain the balloon inside tube (26); and
heat-welding the balloon (5) at a distal side thereof to a distal part of the balloon inside tube (26) at a temperature equal to or higher than the melting point of the thermoplastic polymer forming the inner layer (21) of the balloon inside tube (26), wherein the thermoplastic polymer forming the inner layer (21) has a higher melting point than the thermoplastic polymer forming the outer layer (22).

16. The method according to claim 15, further comprising the steps of:
swaging a radiopaque marker (24) onto the outer surface of the inner tube (26); and
disposing the support body (23) on the outer surface of the inner tube (26) and an outer surface of the radiopaque marker (24).

## Patentansprüche

1. Ballonkatheter (A; A'), mit:
einem Ballon (5); und
einer Balloninnenröhre (26), die durch ein inneres Lumen des Ballons (5) geht, wobei die Balloninnenröhre (26) eine innere Schicht (21), eine äußere Schicht (22) und einen Stützkörper (23) aufweist,
wobei der Ballon (5), die innere Schicht (21) und die äußere Schicht (22) aus thermoplastischen Polymeren ausgebildet sind, die miteinander wärmeverschweißt werden können, und
ein thermoplastisches Polymer, das die innere Schicht (21) ausbildet, einen höheren Schmelzpunkt als ein thermoplastisches Polymer hat, das die äußere Schicht (22) ausbildet;
**dadurch gekennzeichnet, dass**
der Ballon (5) an einer distalen Seite davon eine distalseitige Manschette (7) hat und die distalseitige Manschette (7) mit einem distalen Teil der Balloninnenröhre (26) bei einer Temperatur wärmeverschweißt ist, die gleich oder höher als der Schmelzpunkt des thermoplastischen Polymers ist, das die innere Schicht (21) der Balloninnenröhre (26) ausbildet.

2. Ballonkatheter (A; A') nach Anspruch 1, wobei die Balloninnenröhre (26) ausgebildet ist, indem die äußere Schicht (22) mit der inneren Schicht (21) und dem Stützkörper (23) bei einer Temperatur wärmeverschweißt wird, die gleich oder höher als der Schmelzpunkt des thermoplastischen Polymers, das die äußere Schicht (22) ausbildet, sowie niedriger als der Schmelzpunkt des thermoplastischen Polymers ist, das die innere Schicht (21) ausbildet.

3. Ballonkatheter (A; A') nach Anspruch 1 oder 2, wobei der Ballon (5) an einer proximalen Seite davon eine proximalseitige Manschette (8) aufweist und die proximalseitige Manschette (8) mit einem proximalen Teil der Balloninnenröhre (26) bei einer Temperatur wärmeverschweißt ist, die gleich oder höher als der Schmelzpunkt des thermoplastischen Polymers ist, das die innere Schicht (21) der Balloninnenröhre (26) ausbildet.

4. Ballonkatheter (A; A') nach einem der Ansprüche 1 bis 3, wobei jedes der thermoplastischen Polymere ein Polyamid oder ein Polyamid-Elastomer ist.

5. Ballonkatheter (A; A') nach einem der Ansprüche 1 bis 4, wobei die Shore-D-Härte der inneren Schicht (21) in dem Bereich von 63D bis 74D ist.

6. Ballonkatheter (A; A') nach einem der Ansprüche 1 bis 5, wobei die Shore-D-Härte der äußeren Schicht (22) in dem Bereich von 40D bis 55D ist.

7. Ballonkatheter (A; A') nach einem der Ansprüche 1 bis 6, wobei der Stützkörper (23) in der äußeren Schicht (22) eingebettet ist, sich zumindest über einen Teil der Balloninnenröhre (26) erstreckt, wobei der Teil innerhalb des inneren Lumens des Ballons positioniert ist, und sich über die gesamte Umfangsrichtung der Balloninnenröhre (26) erstreckt.

8. Ballonkatheter (A; A') nach Anspruch 7, wobei der Stützkörper (23) ein wendelförmiger Stützkörper (23) ist.

9. Ballonkatheter (A; A') nach Anspruch 8, wobei der wendelförmige Stützkörper (23) aus einem rechteckigen Draht ausgebildet ist.

10. Ballonkatheter (A; A') nach Anspruch 8, wobei der wendelförmige Stützkörper (23) aus einem Runddraht ausgebildet ist.

11. Ballonkatheter (A; A') nach einem der Ansprüche 1 bis 10, der ferner eine strahlenundurchlässige Markierung (24) aufweist, die sich innerhalb der Balloninnenröhre (26) befindet.

12. Ballonkatheter (A; A') nach Anspruch 11, wobei sich die strahlenundurchlässige Markierung (24) in der radialen Richtung weiter innen befindet als der Stützkörper (23).

13. Ballonkatheter (A; A') nach einem der Ansprüche 1 bis 12, wobei die Balloninnenröhre (26) mit einem distalen Ende eines distalseitigen Schafts (4) verbunden ist, ein proximalseitiger Schaft (6) mit einem proximalen Ende des distalseitigen Schafts (4) verbunden ist und eine Muffe (9) an einem proximalen Teil des proximalseitigen Schafts (6) befestigt ist.

14. Ballonkatheter (A; A') nach einem der Ansprüche 1 bis 13, wobei der Ballonkatheter (A; A') ein Perfusionsballonkatheter (A; A') ist und ein inneres Lumen der Balloninnenröhre (26) ein Perfusionslumen ausbildet.

15. Verfahren zum Herstellen eines Ballonkatheters (A; A'), der einen Ballon und eine Balloninnenröhre (26) aufweist, die durch ein inneres Lumen des Ballons (5) geht, wobei die Balloninnenröhre (26) eine innere Schicht (21), eine äußere Schicht (22) und einen Stützkörper (23) aufweist, wobei das Verfahren die folgenden Schritte aufweist:
Einsetzen eines Kernelements (27) in eine Innenröhre (26) zum Ausbilden der inneren Schicht (21) der Balloninnenröhre (26), wobei die Innenröhre (26) aus einem thermoplastischen Polymer ausgebildet ist, das mit dem Ballon (5) wärmeverschweißt werden kann;
Anordnen des Stützkörpers (23) an einer Außenfläche der Innenröhre (26);
Anordnen einer Außenröhre (28) zum Ausbilden der äußeren Schicht (22) der Balloninnenröhre (26), wobei die äußere Röhre (28) aus einem thermoplastischen Polymer ausgebildet ist, das mit dem Ballon (5) wärmeverschweißt werden kann, um den Stützkörper (23) zu bedecken;
Anordnen einer Wärmeschrumpfröhre, um die Außenseite der Außenröhre (28) zu bedecken;
Ausführen des Wärmeverschweißens der äußeren Schicht (22) mit der inneren Schicht (21) und dem Stützkörper (23) bei einer Temperatur, die gleich oder höher als der Schmelzpunkt des thermoplastischen Polymers, das die äußere Schicht (22) ausbildet, sowie niedriger als der Schmelzpunkt des thermoplastischen Polymers ist, das die innere Schicht (21) ausbildet;
Entfernen der Wärmeschrumpfröhre;
Entfernen des Kernelements (27), um die Balloninnenröhre (26) zu erhalten; und
Verschweißen des Ballons (5) an einer distalen Seite davon mit einem distalen Teil der Balloninnenröhre (26) bei einer Temperatur, die gleich oder höher als der Schmelzpunkt des thermoplastischen Polymers ist, das die innere Schicht (21) der Balloninnenröhre (26) ausbildet, wobei das thermoplastische Polymer, das die innere Schicht (21) ausbildet, einen höheren Schmelzpunkt als das thermoplastische Polymer hat, das die äußere Schicht (22) ausbildet.

16. Verfahren nach Anspruch 15, das ferner die folgenden Schritte aufweist:
Aufbringen eines strahlenundurchlässigen Markers (24) an der Außenfläche der Innenröhre (26); und
Anordnen des Stützkörpers (23) an der Außenfläche der Innenröhre (26) und einer Außenfläche der strahlenundruchlässigen Markierung (24).

## Revendications

1. Cathéter à ballonnet (A ; A') comprenant :
un ballonnet (5) ; et
un tube intérieur à ballonnet (26) traversant une lumière interne du ballonnet (5), le tube intérieur à ballonnet (26) comportant une couche intérieure (21), une couche extérieure (22) et un corps de support (23),
dans lequel le ballonnet (5), la couche intérieur (21) et la couche extérieur (22) sont formés de polymères thermoplastiques qui peuvent être thermosoudés l'un à l'autre, et
un polymère thermoplastique formant la couche intérieure (21) a un point de fusion plus élevé qu'un polymère thermoplastique formant la couche extérieure (22) ;
**caractérisé en ce que**
le ballonnet (5) possède un manchon latéral distal (7) sur un côté distal de celui-ci, et le manchon latéral distal (7) est thermosoudé à une partie distale du tube intérieur à ballonnet (26) à une température égale ou supérieure au point de fusion du polymère thermoplastique formant la couche intérieur (21) du tube intérieur à ballonnet (26).

2. Cathéter à ballonnet (A ; A') selon la revendication 1, dans lequel le tube intérieur à ballonnet (26) est formé par thermosoudage de la couche extérieur (22) à la couche intérieur (21) et au corps de support (23) à une température qui est égale ou supérieure au point de fusion du polymère thermoplastique formant la couche extérieur (22) ainsi qu'inférieure au point de fusion du polymère thermoplastique formant la couche intérieur (21).

3. Cathéter à ballonnet (A ; A') selon la revendication 1 ou 2, dans lequel le ballonnet (5) possède un manchon latéral proximal (8) sur un côté proximal de celui-ci, et le manchon latéral proximal (8) est thermosoudé à une partie proximale du tube intérieur à ballonnet (26) à une température égale ou supérieure au point de fusion du polymère thermoplastique formant la couche intérieur (21) du tube intérieur à ballonnet (26).

4. Cathéter à ballonnet (A ; A') selon l'une quelconque des revendications 1 à 3, dans lequel chacun des polymères thermoplastiques est un polyamide ou un élastomère polyamide.

5. Cathéter à ballonnet (A ; A') selon l'une quelconque des revendications 1 à 4, dans lequel la dureté Shore D de la couche intérieur (21) est comprise entre 63D et 74D.

6. Cathéter à ballonnet (A ; A') selon l'une quelconque des revendications 1 à 5, dans lequel la dureté Shore D de la couche extérieur (22) est comprise entre 40D et 55D.

7. Cathéter à ballonnet (A ; A') selon l'une quelconque des revendications 1 à 6, dans lequel le corps de support (23) est incorporé dans la couche extérieur (22), s'étend au moins sur une partie du tube intérieur à ballonnet (26), la partie étant positionnée dans la lumière interne du ballonnet, et s'étend sur toute la direction circonférentielle du tube intérieur à ballonnet (26).

8. Cathéter à ballonnet (A ; A') selon la revendication 7, dans lequel le corps de support (23) est un corps de support en forme de hélice (23).

9. Cathéter à ballonnet (A ; A') selon la revendication 8, dans lequel le corps de support en forme de hélice (23) est formé d'un fil rectangulaire.

10. Cathéter à ballonnet (A ; A') selon la revendication 8, dans lequel le corps de support en forme de hélice (23) est formé d'un fil rond.

11. Cathéter à ballonnet (A ; A') selon l'une quelconque des revendications 1 à 10, comprenant en outre un marqueur radio-opaque (24) situé dans le tube intérieur à ballonnet (26).

12. Cathéter à ballonnet (A ; A') selon la revendication 11, dans lequel le marqueur radio-opaque (24) est situé à une position plus interne dans la direction radiale que le corps de support (23).

13. Cathéter à ballonnet (A ; A') selon l'une quelconque des revendications 1 à 12, dans lequel le tube intérieur à ballonnet (26) est relié à une extrémité distale d'un arbre latéral distal (4), un arbre latéral proximal (6) est relié à une extrémité proximale de l'arbre latéral distal (4), et un moyeu (9) est fixé à une partie proximale de l'arbre latéral proximal (6).

14. Cathéter à ballonnet (A ; A') selon l'une quelconque des revendications 1 à 13, dans lequel le cathéter à ballonnet (A ; A') est un cathéter à ballonnet de perfusion (A ; A'), et une lumière interne du tube intérieur à ballonnet (26) forme une lumière de perfusion.

15. Procédé de fabrication d'un cathéter à ballonnet (A ; A') comportant un ballonnet, et un tube intérieur à ballonnet (26) traversant une lumière interne du ballonnet (5), le tube intérieur à ballonnet (26) comportant une couche intérieur (21), une couche extérieur (22), et un corps de support (23), le procédé comprenant les étapes suivantes :
l'insertion d'un élément central (27) dans un tube intérieur (26) pour former la couche intérieure (21) du tube intérieur à ballonnet (26), le tube intérieur (26) étant formé d'un polymère thermoplastique qui peut être thermosoudé au ballonnet (5) ;
la disposition du corps de support (23) sur une surface extérieure du tube intérieur (26) ;
la disposition d'un tube extérieur (28) pour former la couche extérieure (22) du tube intérieur à ballonnet (26), le tube extérieur (28) étant formé d'un polymère thermoplastique qui peut être thermosoudé au ballonnet (5), de manière à recouvrir le corps de support (23) ;
la disposition d'un tube thermorétractable de manière à recouvrir l'extérieur du tube extérieur (28) ;
l'exécution d'une thermosoudure de la couche extérieure (22) à la couche intérieure (21) et au corps de support (23) à une température qui est égale ou supérieure au point de fusion du polymère thermoplastique formant la couche extérieure (22) ainsi qu'inférieure au point de fusion du polymère thermoplastique formant la couche intérieure (21) ;
l'enlèvement du tube thermorétractable ;
l'enlèvement du élément central (27) pour obtenir le tube intérieur à ballonnet (26) ; et
le thermosoudage du ballonnet (5) à un côté distal de celui-ci à une partie distale du tube intérieur à ballonnet (26) à une température égale ou supérieure au point de fusion du polymère thermoplastique formant la couche intérieure (21) du tube intérieur à ballonnet (26), le polymère thermoplastique formant la couche intérieure (21) ayant un point de fusion plus élevé que le polymère thermoplastique formant la couche extérieure (22).

16. Procédé selon la revendication 15, comprenant en outre les étapes de :
la rétreint d'un marqueur radio-opaque (24) sur la surface extérieure du tube interne (26) ; et
la disposition du corps de support (23) sur la surface extérieure du tube intérieur (26) et sur une surface extérieure du marqueur radio-opaque (24).
